# FASCICULE DE BREVET EUROPEEN

(11) **EP 0 416 983 B1**
(45) Date de publication et mention de la délivrance du brevet: **28.07.1993**
(21) Numéro de dépôt: 90402395.9
(22) Date de dépôt: 30.08.1990
(51) Int. Cl.: A61K 35/16

(54) **Procédé de préparation de concentré du complexe Facteur VIII-facteur von Willebrand de la coagulation sanguine à partir de plasma total**
Verfahren zur Herstellung eines Konzentrats des Faktor VIII-von Willebrand-Faktor-Komplexes der Blutgerinnung aus Vollplasma
Process for the preparation of a concentrate from factor VIII-factor von Willebrand of blood coagulation from the whole plasma

(30) Priorité: 05.09.1989 FR 8911567
(43) Date de publication de la demande: 13.03.1991
(73) Titulaire: CENTRE REGIONAL DE TRANSFUSION SANGUINE DE LILLE, 59012 Lille (FR)
(72) Inventeur: Burnouf-Radosevich, Miryana, F-59136 Wavrin (FR); Burnouf Thierry, F-59136 Wavrin (FR)
(74) Mandataire: Lhuillier, René

(56) Documents cités:
- EP-A- 0 053 046
- EP-A- 0 104 356
- EP-A- 0 176 926
- EP-A- 0 359 593
- FR-A- 2 184 898
- US-A- 4 386 068
- US-A- 4 435 318

## Description

L'invention concerne un procédé de préparation d'un concentré du complexe Facteur VIII - facteur von Willebrand à haute activité spécifique à partir du plasma sanguin total.

Le traitement de l'hémophilie A par l'injection de Facteur VIII est couramment pratiqué et nécessite l'utilisation de préparations de haute pureté, d'une part pour réduire les risques de contamination virale, d'autre part parce que les injections doivent être souvent répétées et que tout excès de matériel contaminant pourrait déclencher des réactions immunitaires indésirables et dangereuses pour le patient.

Plusieurs méthodes sont déjà utilisées dans les centres industriels de traitement du plasma humain pour purifier le Facteur VIII. Ces méthodes comprennent des précipitations des protéines contaminantes par divers agents chimiques, des chromatographies de tamisage moléculaire, des chromatographies d'immunoaffinité, des chromatographies d'échange d'ions et diverses combinaisons de ces différentes méthodes.

Le Facteur VIII étant présent en faible quantité dans le plasma, le rendement des procédés de purification est le principal problème à résoudre. En outre le Facteur VIII est une protéine instable et qui peut être activée par d'autres facteurs sanguins or à l'état activé il perd son intérêt thérapeutique ; les procédés de purification et de dosage final doivent donc être adaptés à ce problème.

La Demanderesse a déjà mis au point une méthode de purification par chromatographie d'échange d'ions qui a été décrite dans la demande de brevet européen 0 359 593 et qui permet d'obtenir un concentré de Facteur VIII de haute pureté.

Toutefois dans ce procédé, comme dans ceux utilisés par d'autres producteurs, on utilise comme matériau de départ une fraction cryoprécipitée du plasma. Cette étape de cryoprécipitation cause une perte de 30 à 40 % du Facteur VIII qui reste dans le surnageant.

Il serait donc avantageux de mettre au point un procédé de préparation à partir du plasma total non cryoprécipité pour limiter les pertes en Facteur VIII. En outre un tel procédé représenterait une simplification très avantageuse pour des centres de production mal équipés où la cryoprécipitation peut être difficile à réaliser.

C'est pourquoi la Demanderesse a mis au point un procédé simple de purification du Facteur VIII à partir du plasma total qui assure l'obtention d'un concentré de haute pureté et stable, avec un très bon rendement.

L'invention concerne donc un procédé de préparation d'un concentré de Facteur VIII à partir d'un plasma total, procédé qui comprend une prépurification destinée à éliminer les constituants du complexe prothrombinique ( Facteurs II, VII, IX, X ) et une purification par chromatographie d'échange d'anions qui, par le choix du gel et du tampon d'élution permet d'obtenir un concentré du complexe Facteur VIII -facteur von Willebrand de haute pureté.

Le procédé permet également d'obtenir, après une étape de purification supplémentaire, des solutions purifiées des autres protéines du plasma comme le fibrinogène, la fibronectine, l'albumine, les immunoglobulines et l'antithrombine III.

Le procédé a été mis au point sur du plasma humain mais il est également applicable à un plasma d'origine animale.

Le procédé selon la présente invention permet donc d'utiliser comme matériau de départ un plasma total, c'est à dire un plasma frais ou congelé pour sa conservation, mais non cryoprécipité. Ce plasma aura été avantageusement récolté en présence de solution anticoagulante ou de stabilisant. Classiquement on utilise un mélange citrate-dextrose-phosphate ; tout stabilisant spécifique du Facteur VIII pourra avantageusement y être ajouté ou substitué.

Comme solution stabilisante du plasma de départ on utilisera avantageusement un mélange d'héparine à 0,2 à 2 U/ml, d'EDTA à 1 à 5 mM, de CaCl₂ 1 à 10 mM, éventuellement additionné de glucose à une concentration comprise entre 5 et 60 g/l.

Le procédé selon la présente invention comprend une première étape de prépurification qui combine une précipitation au chlorure de baryum et une adsorption sur gel d'hydroxyde d'aluminium.

Un procédé de purification du Facteur VIII comprenant une adsorption sur hydroxyde d'aluminium suivie d'une ultrafiltration sur fibres creuses a déjà été décrit dans le brevet US-A-4 386 068. L'emploi de sels de baryum a déjà été décrit dans le brevet US-A-4 435 318. Toutefois la Demanderesse a observé qu'on obtenait des résultats particulièrement satisfaisants par la combinaison de ces deux traitements, alors qu'un traitement à l'alhydrogel seul ne permet pas d'éliminer complètement la prothrombine et les autres composants du PPSB et qu'un traitement au chlorure de baryum seul laisse subsister du Facteur X, de la prothrombine et surtout du Facteur VII.

Le traitement au chlorure de baryum est avantageusement réalisé sur le plasma dont le pH est ajusté à 6,5, par addition d'une solution 1 M en chlorure de baryum jusqu'à obtention d'une concentration finale de 0,08 M, sous agitation, et est suivi d'une centrifugation à 5° à 10°C destinée à éliminer les protéines précipitées, puis de la récupération du surnageant. Les protéines précipitées pourront avantageusement être récupérées pour la production du complexe prothrombinique ou de ses constituants.

Le surnageant est ensuite mis en présence de gel d'hydroxyde d'aluminium à 3 %, à pH 6,5, qui adsorbe les protéines contaminantes résiduelles ; ce traitement est suivi d'un refroidissement à 5° à 8°C en cryostat, d'une centrifugation à 5°C et de la récupération du surnageant, qui est maintenu à 5° à 8°C.

Ce surnageant doit subir un dessalage qui peut être effectué soit par ultrafiltration en présence du tampon d'équilibrage de la chromatographie suivante additionné d'héparine à 0,5 à 2 U/ml, soit par chromatographie sur Sephadex® G25 dans le même tampon.

Le procédé selon la présente invention comprend ensuite une séparation par chromatographie d'échange d'anions. La Demanderesse a déjà décrit dans la demande de brevet EP-A-0 359 593, les avantages qu'elle a mis en évidence d'un type de résine qui offre un faible pouvoir d'échange ionique et qui présente des pores de grande dimension, ceux-ci permettant la rétention de molécules de très grande taille, c'est-à-dire d'un poids moléculaire pouvant être supérieur à 1 million de daltons. Cette rétention prolongée permet l'établissement de liaisons de caractère faiblement hydrophobe avec la résine et le choix de la force ionique du tampon permet une désorption sélective des molécules fixées.

Des résines de ce type ont été décrites pour la première fois par Y. KATO et al. (J. Chromato. 245, 1982, 193-211) qui recommandaient leur utilisation pour des séparations chromatographiques, à moyenne performance et à l'échelle industrielle, de protéines de très grande taille.

Des résines de ce type sont disponibles dans le commerce sous l'appellation générique Fractogel 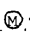 . On peut ainsi utiliser le DEAE-Fractogel 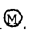 650 et le T- ou D-MAE-Fractogel 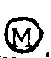 (Merck). Ces mêmes résines sont actuellement disponibles sous une forme qualifiée par le fournisseur de "résines de type tentaculaire". La structure de leur matrice est modifiée afin d'augmenter la surface de fixation des charges positives ce qui peut favoriser une augmentation de la capacité du gel.

Le tampon d'équilibrage de la chromatographie est un tampon à base de citrate de sodium et de chlorure de sodium, ajusté à pH 7, qui contient avantageusement du chlorure de calcium à une concentration comprise entre 0,5 et 6 mM et de la lysine à une concentration de l'ordre de 2 à 4 g/l et du glycocolle à une concentration de 8 à 11 g/l. La mise en oeuvre du procédé comprendra des augmentations définies de cette concentration en chlorure de sodium.

La mise en oeuvre du procédé de purification selon l'invention comprend l'injection de la préparation prépurifiée décrite plus haut sur la colonne de chromatographie. Dans les conditions définies (0,11 M NaCl), la colonne peut fixer les molécules de très grande taille comme le complexe facteur von Willebrand-Facteur VIII et laisse passer dans le filtrat le fibrinogène, l'albumine, les immunoglobulines, l'antithrombine III et la fibronectine.

Selon un mode de réalisation préféré de l'invention qui vise à obtenir le meilleur rendement de récupération du Facteur VIII, la force ionique du tampon d'élution de la chromatographie est augmentée une seule fois, à 0,27 M de chlorure de sodium. Selon un autre mode de réalisation de l'invention cette élution est précédée par un prélavage par augmentation de la force ionique à 0,13 M de chlorure de sodium, ce qui élimine la fibronectine.

Le complexe Facteur VIII-facteur von Willebrand désorbé et élué dans ces conditions présente une activité spécifique au moins égale à 5 à 10 U/mg. Le rendemment global du procédé est au moins égal à 350 U/litre de plasma initial et peut être au moins égal à 500 U/litre quand le plasma initial est additionné du mélange stabilisant décrit plus haut.

En fonction de son utilisation ultérieure, cette solution de complexe FacteurVIII-facteur von Willebrand peut encore être soumise à une étape supplémentaire de purification et particulièrement de concentration par une nouvelle chromatographie. Celle-ci pourra être effectuée, comme la précédente, sur DEAE- ou T/D-MAE-Fractogel® ou sur d'autres résines; d'autres supports comme le sulfate de dextran, l'amino-hexyl immobilisé, l'héparine immobilisée, les résines de sulfopropyle, d'affinité ou d'immunoaffinité peuvent aussi être utilisés.

Cette chromatographie supplémentaire est effectuée dans le même tampon de base que la précédente. Selon un mode de réalisation préféré de l'invention qui vise à obtenir du Facteur VIII le plus concentré possible, cette chromatographie supplémentaire est effectuée dans les mêmes conditions que la première, c'est-à-dire avec une seule étape de désorption par augmentation de la force ionique du tampon à 0,27 M NaCl. Selon un autre mode de réalisation de l'invention, par une première augmentation de la force ionique du tampon à 0,13 M on élimine les protéines contaminantes résiduelles et par une seconde augmentation à 0,27 M on récupère le complexe Facteur VIII -facteur von Willebrand concentré de haute pureté, qui présente alors une activité spécifique au moins égale à 10 à 20 U/mg.

Les autres protéines du premier filtrat de la colonne comme les immunoglobulines, l'albumine, l'antithrombine III, le fibrinogène et la fibronectine peuvent également être purifiées et concentrées par des méthodes chromatographiques classiques.

Le procédé selon la présente invention comprend également un traitement d'inactivation virale selon une technique connue. Dans le cas où on utilise un agent chimique, par exemple un traitement par solvant-détergent, il sera judicieux d'effectuer ce traitement avant l'une quelconque des étapes chromatographiques pour que celle-ci assure l'élimination des agents d'inactivation.

Les concentrés du complexe Facteur VIII-facteur von Willebrand et des autres protéines plasmatiques obtenus par le procédé décrit sont aussi l'objet de la présente invention.

Lesdits concentrés sont conditionnés selon les normes de la pharmacopée et peuvent être destinés à un usage thérapeutique.

Les exemples suivants illustrent deux modes de réalisation de l'invention sans toutefois en limiter la portée.

### EXEMPLE 1

On utilise 250 ml de plasma frais ou congelé-décongelé à 22-25°C, récolté en présence d'anticoagulant/stabilisant (citrate-dextrose-phosphate, par exemple), et ajusté à un pH de 6,5 par de l'acide acétique

### a) Prépurification

On y ajoute 20 ml de chlorure de baryum 1 M à pH 6,5 au moyen d'une pompe péristaltique jusqu'à obtention d'une concentration finale de 0,08 M. L'addition se fait à un débit de 4 à 8 ml par minute puis le mélange est laissé sous agitation, à température ordinaire, pendant 15 minutes.

Le mélange est ensuite centrifugé à 2.700 tours/minute pendant 20 minutes, à 8°C, puis le surnageant est récupéré.

Le surnageant est ensuite adsorbé sur un gel d'hydroxyde d'aluminium (Alhydrogel Eurobio à 3 % d'Al(OH)₃) à raison de 2,3 g/l de plasma. Le pH est ajusté à 6,5 et la température est abaissée jusqu'à 5°C en cryostat. Le mélange est centrifugé à 5°C à 2700 tours/minute pendant 20 minutes et le surnageant est récupéré et maintenu à 5°C.

Ce traitement permet l'élimination des composants du complexe prothrombinique (Facteurs II, VII, IX, X) qui peuvent être récupérés et purifiés selon des procédés connus.

On obtient des résultats particulièrement satisfaisants par la combinaison de ces deux traitements, alors qu'un traitement à l'alhydrogel seul ne permet pas d'éliminer complètement la prothrombine et les autres composants du PPSB et qu'un traitement au chlorure de baryum seul laisse subsister du Facteur X, de la prothombine et surtout du Facteur VII.

Le surnageant récolté est dessalé soit par ultrafiltration en présence du tampon de la chromatographie suivante (voir plus bas) additionné de 1 U/ml d'héparine, soit par chromatographie sur Sephadex® G25, dans le même tampon.

Un traitement d'inactivation virale classique au solvant-détergent est ensuite appliqué, pendant 6 heures à 24°C (Cette méthode de traitement a été décrite dans la demande de brevet européen n°0 131 740).

### b) Purification par chromatographie

Le surnageant prépurifié et dialysé est soumis à une étape de purification par chromatographie. On utilise une colonne K26/30 (Pharmacia-Uppsala-Suède) d'un diamètre de 2,6 cm et d'une hauteur utile de 30 cm, remplie sur 10 cm par la résine DEAE-Fractogel-TSK 650(M) (Merck).

Le tampon d'équilibrage de la colonne et de charge de l'échantillon a la composition suivante : citrate de sodium 10 mM, chlorure de calcium 1mM, glycocolle 9 g/l, lysine 3 g/l. Ce tampon est additionné de chlorure de sodium, ajusté à une concentration finale de 0,11 M et à un pH de 7.
L'échantillon est injecté sur la colonne à un débit de 100 ml/h.

La colonne est lavée avec le tampon d'équilibrage pour éliminer les protéines non adsorbées, qui comprennent le fibrinogène, l'albumine, les immunoglobulines, l'antithrombine III et la fibronectine ainsi que les agents d'inactivation virale.

Le complexe FacteurVIII-facteur von Willebrand est ensuite désorbé par augmentation de la force ionique du tampon à 0,27 M de chlorure de sodium.

La solution de Facteur VIII obtenue par ce procédé présente une activité spécifique de 5 à 10 UI/mg et le rendement de la purification par rapport au plasma injecté sur la colonne est de 60 à 80 %.

On observe toujours un rapport proche de 1U/1U pour la concentration des deux facteurs VIII et von Willebrand, ce dernier étant exprimé en unités de cofacteur de la ristocétine.

La pureté de cette solution de Facteur VIII peut encore être légèrement améliorée par une étape supplémentaire de séparation chromatographique qui permet de concentrer le produit. On utilise par exemple une seconde colonne de DEAE-Fractogel, dans les mêmes conditions de charge que précédemment, et on effectue un prélavage à 0,13 M de NaCl, ce qui élimine les protéines contaminantes résiduelles. La force ionique du tampon est ensuite augmentée à 0,27 M de chlorure de sodium pour désorber et éluer le complexe Facteur VIII-facteur von Willebrand hautement purifié et concentré.

La deuxième étape de concentration par chromatographie peut être remplacée par une ultrafiltration.

Selon le mode de réalisation habituel de la présente invention, la première purification par chromatographie est réalisée sur une résine de DEAE-Fractogel®. Toutefois de très bons résultats ont également été obtenus avec les nouvelles résines commercialisées par Merck, comme le TMAE-Fractogel® (TMAE = Tri. Méthyl. Amino. Ethyl) ou le DMAE-Fractogel® (DMAE = Di-MAE), qui présentent des propriétés équivalentes à celles du DEAE-Fractogel®. On peut également utiliser les nouvelles résines de type "tentaculaire" mises au point par la société Merck et présentées par W. Müller à la "Conference on Liquid chromatography", juin 1989, à Stockholm.

### EXEMPLE 2

Un autre mode de réalisation de la présente invention permet également de préparer des concentrés de fibrinogène et de fibronectine tout en fournissant un Facteur VIII-facteur von Willebrand concentré avec un rendement plus faible et une activité spécifique légèrement améliorée.

Le procédé est identique à celui de l'exemple 1 jusqu'à l'élution du FacteurVIII-facteur von Willebrand de la première colonne de DEAE-Fractogel®.

Le fibrinogène, l'antithrombine III, l'albumine et les immunoglobulines ne sont pas retenus par la colonne et peuvent être récupérés dans le filtrat.

Le complexe Facteur VIII-facteur von Willebrand peut être purifié et concentré par une deuxième séparation chromatographique sur une colonne de DEAE-Fractogel®, avec le tampon d'équilibrage à une force ionique de 0,17 M de chlorure de sodium pour ne pas fixer des protéines contaminantes résiduelles ; une augmentation de la force ionique à 0,27 M de chlorure de sodium permet de désorber et éluer le complexe Facteur VIII-facteur von Willebrand. On obtient ainsi une activité spécifique de 10 à 20 IU/mg.

Le fibrinogène et la fibronectine peuvent ensuite être purifiés et concentrés, selon des méthodes chromatographiques connues, pour donner des solutions d'une qualité conforme pour un usage thérapeutique et qui ont été décrites dans la demande de brevet français n° 88 07530.

Les autres protéines du plasma peuvent être purifiées-concentrés selon des méthodes classiques de fractionnement.

### EXEMPLE 3

On peut encore améliorer le rendement de la purification en stabilisant le plasma de départ, dès sa décongélation à 25°C, par l'addition du mélange suivant :
héparine 1 U/ml
EDTA 2 mM, soit 0,74 g/l
chlorure de calcium 6 mM, soit 0,67 g/l

On peut encore ajouter à ce mélange du glucose à une concentration comprise entre 5 et 60 g/l.

Le pH est ensuite abaissé à 6,5 par l'addition d'acide acétique.

Les étapes de purifications sont ensuite appliquées comme dans l'exemple 1 ou 2.

Dans ces conditions, le rendement global du procédé de purification est au-moins égal à 500 U de FVIII:C/litre de plasma initial.

Ceci correspond à une récupération globale de 55 à 65% de l'activité de Facteur VIII pour une activité spécifique comprise entre 10 et 30 unités de FVIII:C/mg de protéine.

## Revendications

1. Procédé de préparation d'un concentré de Facteur VIII-facteur von Willebrand stable et à haute activité spécifique caractérisé en ce qu'on soumet un plasma total à une prépurification par un double traitement qui comprend une précipitation au chlorure de baryum et une adsorption sur gel d'hydroxyde d'aluminium, et à une purification par chromatographie sur un gel échangeur d'anions permettant la rétention de molécules de très grande taille.

2. Procédé selon la revendicaion 1 caractérisé en ce que le plasma total est un plasma frais ou congelé.

3. Procédé selon la revendication 1 ou 2 caractérisé en ce que le plasma est additionné d'un mélange stabilisant comprenant de l'héparine à 0,2 à 2 U/ml, de l'EDTA à 1 à 5 mM et du CaCl₂ à 1 à 10 mM.

4. Procédé selon la revendication 3, caractérisé en ce que le mélange stabilisant comprend, en outre du glucose à une concentration comprise entre 5 et 60 g/l.

5. Procédé selon l'une quelconque des revendications 1 à 4 caractérisé en ce que la prépurification comprend :
a) une précipitation au chlorure de baryum suivie d'une centrifugation et de la récupération du surnageant,
b) une adsorption sur gel d'hydroxyde d'aluminium suivie d'une centrifugation à froid et de la récupération du surnageant,
c) un traitement de dessalage.

6. Procédé selon la revendication 5 caractérisé en ce que la précipitation au chlorure de baryum est effectuée par addition d'une solution de chlorure de baryum 1 M à pH6,5, sous agitation, et est suivie d'une centrifugation à 5 à 10° C et de la récupération du surnageant.

7. Procédé selon la revendication 5 ou 6 caractérisé en ce que l'adsorption sur gel d'hydroxyde d'aluminium est réalisée avec un gel à 3%, à un pH de 6,5 et est suivie d'un refroidissement rapide à 5°C, d'une centrifugation à 5°C et de la récupération du surnageant.

8. Procédé selon l'une quelconque des revendications 5 à 7 caractérisé en ce que le traitement de dessalage est effectué par ultrafiltration en présence du tampon d'équilibrage de la chromatographie suivante, additionné d'héparine.

9. Procédé selon l'une quelconque des revendications 5 à 7 caractérisé en ce que le traitement de dessalage est effectué par chromatographie sur colonne de gel de filtration simple, comme le Séphadex 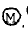 G 25, en tampon d'équilibrage de la chromatographie suivante, additionné d'héparine.

10. Procédé selon l'une quelconque des revendications 1 à 9 caractérisé en ce que le surnageant du plasma ayant subi une prépurification est injecté sur une colonne de chromatographie renfermant un gel échangeur d'anions qui n'exclut pas les molécules de très grande taille et laisse passer dans le filtrat le fibrinogène, l'albumine, les immunoglobulines, l'antithrombine III et la fibronectine.

11. Procédé selon l'une quelconque des revendications 1 à 10 caractérisé en ce que le gel de chromatographie est un gel de type polymère vinylique greffé de groupements de type DEAE ou T- ou D-MAE.

12. Procédé selon la revendication 11 caractérisé en ce que le gel de polymère est du Fractogel 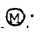 .

13. Procédé selon l'une quelconque des revendications 10 à 12 caractérisé en ce que le tampon d'équilibrage de la chromatographie est un tampon à base de citrate de sodium et de chlorure de calcium, qui contient du glycocolle et de la lysine, qui est additionné de chlorure de sodium 0,11 M et est ajusté à pH 7.

14. Procédé selon la revendication 13 caractérisé en ce que le tampon contient 8 à 11 g/l de glycocolle et 2 à 4 g/l de lysine.

15. Procédé selon l'une quelconque des revendications 10 à 14 caractérisé en ce que la force ionique du tampon est augmentée à 0,27 M de chlorure de sodium pour désorber le complexe Facteur VIII-facteur von Willebrand de la colonne de chromatographie.

16. Procédé selon l'une quelconque des revendications 10 à 14 caractérisé en ce que la force ionique du tampon est augmentée à 0,13 M de chlorure de sodium ce qui élimine la fibronectine puis à 0,27 M de chlorure de sodium pour désorber le complexe Facteur VIII-facteur von Willebrand de la colonne de chromatographie.

17. Procédé selon l'une quelconque des revendications 1 à 16 caractérisé en ce que le complexe Facteur VIII-facteur von Willebrand désorbé de la colonne de chromatographie est soumis à une étape supplémentaire de purification-concentration par chomatographie.

18. Procédé selon l'une quelconque des revendications 1 à 17 caractérisé en ce que la chromatographie supplémentaire est effectuée sur une résine échangeuse d'ions choisie parmi le DEAE- ou T/D-MAE-Fractogel 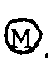 , l'amino-hexyl immobilisé, l'héparine immobilisée, le sulfate de dextran, le sulfopropyle, ou sur résine d'affinité ou d'immunoaffinité.

19. Procédé selon la revendication 17 ou 18 caractérisé en ce que la chromatographie supplémentaire est effectuée en tampon ajusté à 0,11 M de chlorure de sodium et comprend 2 augmentations successives de la force ionique à 0,13 M puis à 0,27 M.

20. Procédé selon la revendication 17 ou 18 caractérisé en ce que la chromatographie supplémentaire est effectuée en tampon ajusté à 0,17 M de chlorure de sodium et comprend une seule augmentation de la force ionique à 0,27 M.

21. Procédé selon l'une quelconque des revendications 1 à 14 caractérisé en ce que les protéines du filtrat de la chromatographie selon la revendication 10, sont soumises à une étape supplémentaire de purification-concentration.

22. Procédé selon l'une quelconque des revendications 1 à 21 caractérisé en ce qu'on effectue un traitement classique d'inactivation virale par solvant-détergent avant l'une quelconque des étapes de chromatographie.

## Patentansprüche

1. Verfahren zur Herstellung eines stabilen Konzentrats von Faktor VIII-von Willebrand-Faktor mit hoher spezifischer Aktivität, **dadurch gekennzeichnet,** daß ein Vollplasma einer Vorreinigung durch eine zweifache Behandlung, welche eine Fällung mit Bariumchlorid und eine Adsorption an Aluminiumhydroxidgel umfaßt und einer Reinigung durch Chromatographie über ein Anionenaustauschergel, welches die Zurückhaltung von sehr großen Molekülen ermöglicht, unterworfen wird.

2. Verfahren nach Anspruch 1, **dadurch gekennzeichnet**, daß das Vollplasma ein frisches oder gefrorenes (congelé) Plasma ist.

3. Verfahren nach Anspruch 1 oder 2, **dadurch gekennzeichnet,** daß dem Plasma ein stabilisierendes Gemisch zugegeben wird, welches 0,2 bis 2 U/ml Heparin, 1 bis 5 mM EDTA und 1 bis 10 mM CaCl₂ umfaßt.

4. Verfahren nach Anspruch 3, **dadurch gekennzeichnet**, daß das stabilisierende Gemisch darüberhinaus Glucose mit einer Konzentration zwischen 5 und 60 g/l umfaßt.

5. Verfahren nach einem der Ansprüche 1 bis 4, **dadurch gekennzeichnet**, daß die Vorreinigung umfaßt:
a) eine Fällung mit Bariumchlorid gefolgt von einer Zentrifugation und der Rückgewinnung des Überstands,
b) eine Adsorption an Aluminiumhydroxidgel gefolgt von einer Zentrifugation in der Kälte und der Rückgewinnung des Überstands,
c) eine Entsalzungsbehandlung.

6. Verfahren nach Anspruch 5, **dadurch gekennzeichnet**, daß die Fällung mit Bariumchlorid durch Zugabe einer Lösung von 1 M Bariumchlorid bei pH 6,5 unter Rühren bewirkt wird und darauf eine Zentrifugation bei 5 bis 10°C und die Rückgewinnung des Überstands folgt.

7. Verfahren nach Anspruch 5 oder 6, **dadurch gekennzeichnet**, daß die Adsorption an Aluminiumhydroxidgel mit einem 3%igen Gel bei einem pH von 6,5 durchgeführt wird und darauf eine rasche Abkühlung auf 5°C, eine Zentrifugation bei 5°C und die Rückgewinnung des Überstands folgt.

8. Verfahren nach einem der Ansprüche 5 bis 7, **dadurch gekennzeichnet**, daß die Entsalzungsbehandlung durch Ultrafiltration in Gegenwart des Equilibrierungspuffers der nachfolgenden Chromatographie, dem Heparin zugegeben ist, bewirkt wird.

9. Verfahren nach einem der Ansprüche 5 bis 7, **dadurch gekennzeichnet**, daß die Entsalzungsbehandlung durch Chromatographie über eine einfache Gelfiltrationssäule, wie Sephadex® G 25, in Equilibrierungspuffer der nachfolgenden Chromatographie, dem Heparin zugegeben ist, bewirkt wird.

10. Verfahren nach einem der Ansprüche 1 bis 9, **dadurch gekennzeichnet,** daß der Überstand des Plasmas, welches vorgereinigt worden ist, auf eine Chromatographiesäule eingespritzt wird, die ein Anionenaustauschergel enthält, welches die sehr großen Moleküle nicht ausschließt und in das Filtrat das Fibrinogen, das Albumin, die Immunglobuline, das Antithrombin III und das Fibronectin durchgehen läßt.

11. Verfahren nach einem der Ansprüche 1 bis 10, **dadurch gekennzeichnet**, daß das Chromatographiegel ein Gel vom Typ eines mit Gruppen vom DEAE- oder T- oder D-MAE-Typ gepfropften Vinylpolymers ist.

12. Verfahren nach Anspruch 11, **dadurch gekennzeichnet**, daß das Polymergel aus Fractogel® ist.

13. Verfahren nach einem der Ansprüche 10 bis 12, **dadurch gekennzeichnet**, daß der Equilibrierungspuffer der Chromatographie ein Puffer auf der Grundlage von Natriumcitrat und Calciumchlorid ist, welcher Glycocoll und Lysin enthält, dem 0,11 M Natriumchlorid zugegeben ist und der auf pH 7 eingestellt ist.

14. Verfahren nach Anspruch 13, **dadurch gekennzeichnet,** daß der Puffer 8 bis 11 g/l Glycocoll und 2 bis 4 g/l Lysin enthält.

15. Verfahren nach einem der Ansprüche 10 bis 14, **dadurch gekennzeichnet**, daß die Ionenstärke des Puffers auf 0,27 M Natriumchlorid erhöht wird, um den Faktor VIII-von Willebrand-Faktor-Komplex von der Chromatographiesäule zu desorbieren.

16. Verfahren nach einem der Ansprüche 10 bis 14, **dadurch gekennzeichnet**, daß die Ionenstärke des Puffers auf 0,13 M Natriumchlorid erhöht wird, was das Fibronectin eliminiert, und danach auf 0,27 M Natriumchlorid erhöht wird, um den Faktor VIII-von Willebrand-Faktor-Komplex von der Chromatographiesäule zu desorbieren.

17. Verfahren nach einem der Ansprüche 1 bis 16, **dadurch gekennzeichnet**, daß der von der Chromatographiesäule desorbierte Faktor VIII-von Willebrand-Faktor-Komplex einem zusätzlichen Reinigungs-Konzentrations-Schritt durch Chromatographie unterworfen wird.

18. Verfahren nach einem der Ansprüche 1 bis 17, **dadurch gekennzeichnet**, daß die zusätzliche Chromatographie über ein Ionenaustauscherharz, das ausgewählt wird unter dem DEAE- oder T/D-MAE-Fractogel®, immobilisiertem Aminohexyl, immobilisiertem Heparin, Dextransulfat, Sulfopropyl, oder über ein Affinitäts- oder Immunaffinitätsharz durchgeführt wird.

19. Verfahren nach Anspruch 17 oder 18, **dadurch gekennzeichnet**, daß die zusätzliche Chromatographie in einem Puffer durchgeführt wird, der auf 0,11 M Natriumchlorid eingestellt ist, und zwei aufeinanderfolgende Erhöhungen der Ionenstärke auf 0,13 M und danach auf 0,27 M umfaßt.

20. Verfahren nach Anspruch 17 oder 18, **dadurch gekennzeichnet**, daß die zusätzliche Chromatographie in einem Puffer durchgeführt wird, der auf 0,17 M Natriumchlorid eingestellt ist, und eine einzige Erhöhung der Ionenstärke auf 0,27 M umfaßt.

21. Verfahren nach einem der Ansprüche 1 bis 14, **dadurch gekennzeichnet**, daß die Proteine des Filtrats der Chromatographie nach Anspruch 10 einem zusätzlichen Reinigungs-Konzentrations -Schritt unterworfen werden.

22. Verfahren nach einem der Ansprüche 1 bis 21, **dadurch gekennzeichnet**, daß eine klassische Behandlung zur Inaktivierung von Viren durch Lösungsmittel-Detergens vor irgendeinem der Chromatographieschritte durchgeführt wird.

## Claims

1. Process for preparing a stable concentrate of the Factor VIII-von Willebrand factor having high specific activity, characterised in that a total plasma is subjected to pre-purification by a double treatment which comprises précipitation with barium chloride and adsorption on aluminium hydroxide gel and to purification by chromatography on an anion exchange gel permitting the retention of very large molecules.

2. Process according to claim 1, characterised in that the total plasma is a fresh or frozen plasma.

3. Process according to claim 1 or 2, characterised in that a stabilising mixture comprising from 0.2 to 2 U/ml of heparin, from 1 to 5 mM EDTA and from 1 to 10 mM CaCl₂ is added to the plasma.

4. Process according to claim 3, characterised in that the stabilising mixture further comprises glucose in a concentration of from 5 to 60 g/l.

5. Process according to any one of claims 1 to 4, characterised in that the pre-purification comprises:
a) precipitation with barium chloride followed by centrifugation and recovery of the supernatant,
b) adsorption on aluminium hydroxide gel followed by cold centrifugation and recovery of the supernatant,
c) desalting treatment.

6. Process according to claim 5, characterised in that the precipitation with barium chloride is effected by adding a solution of 1 M barium chloride at a pH of 6.5, with stirring, and is followed by centrifugation at from 5 to 10°C and recovery of the supernatant.

7. Process according to claim 5 or 6, characterised in that the adsorption on aluminium hydroxide gel is performed with a 3% gel at a pH of 6.5 and is followed by rapid cooling to 5°C, centrifugation at 5°C and recovery of the supernatant.

8. Process according to any one of claims 5 to 7, characterised in that the desalting treatment is performed by ultrafiltration in the presence of the equilibration buffer for the following chromatography step, to which heparin has been added.

9. Process according to any one of claims 5 to 7, characterised in that the desalting treatment is performed by chromatography on a simple filtration gel column, such as Sephadex® G 25, in the equilibration buffer for the following chromatography step, to which heparin has been added.

10. Process according to any one of claims 1 to 9, characterised in that the supernatant of the plasma that has undergone pre-purification is injected onto a chromatography column containing an anion exchange gel which does not exclude very large molecules and allows the fibrinogen, albumin, immunoglobulins, antithrombin III and fibronectin to flow into the filtrate.

11. Process according to any one of claims 1 to 10, characterised in that the chromatography gel is a gel of the vinyl polymer type to which groups of the DEAE or T- or D-MAE type are attached.

12. Process according to claim 11, characterised in that the polymer gel is Fractogel®.

13. Process according to any one of claims 10 to 12, characterised in that the chromatography equilibration buffer is a sodium citrate and calcium chloride based buffer, containing glycine and lysine, to which is added 0.11 M sodium chloride and which is adjusted to a pH of 7.

14. Process according to claim 13, characterised in that the buffer contains from 8 to 11 g/l of glycine and from 2 to 4 g/l of lysine.

15. Process according to any one of claims 10 to 14, characterised in that the ionic strength of the buffer is increased to 0.27 M sodium chloride to desorb the Factor VIII-von Willebrand factor complex from the chromatography column.

16. Process according to any one of claims 10 to 14, characterised in that the ionic strength of the buffer is increased to 0.13 M sodium chloride, which eliminates the fibronectin, and then to 0.27 M sodium chloride to desorb the Factor VIII-von Willebrand factor complex from the chromatography column.

17. Process according to any one of claims 1 to 16, characterised in that the Factor VIII-von Willebrand factor complex desorbed from the chromatography column is subjected to an additional step of purification and concentration by chromatography.

18. Process according to any one of claims 1 to 17, characterised in that the additional chromatography is carried out on an ion exchange resin chosen from the following: DEAE- or T/D-MAE-Fractogel®, immobilised amino-hexyl, immobilised heparin, dextran sulphate, sulphopropyl, or on an affinity or immune-affinity resin.

19. Process according to claim 17 or 18, characterised in that the additional chromatography is performed with a buffer adjusted to 0.11 M sodium chloride and comprises 2 successive increases in ionic strength to 0.13 M and then to 0.27 M.

20. Process according to claim 17 or 18, characterised in that the additional chromatography is performed with a buffer adjusted to 0.17 M sodium chloride and comprises a single increase in ionic strength to 0.27 M.

21. Process according to any one of claims 1 to 14, characterised in that the proteins present in the chromatography filtrate according to claim 10 are subjected to an additional purification and concentration step.

22. Process according to any one of claims 1 to 21, characterised in that a conventional viral inactivation treatment is performed using solvent-detergent prior to any of the chromatography steps.
